# EUROPEAN PATENT APPLICATION

(11) **EP 4 183 424 A1**
(43) Date of publication of application: **24.05.2023**
(21) Application number: 20958690.8
(22) Date of filing: 22.10.2020
(51) Int. Cl.: A61L 15/40, A61L 27/00, A61L 27/36, A61P 17/02

(54) **WOUND CONTACT MEMBER**

(71) Applicant: National University Corporation University Of Toyama, Toyama-shi, Toyama 930-8555 (JP); SAKURA SEIKI CO., LTD., Chikuma-shi Nagano 387-0015 (JP)
(72) Inventor: AMANO Kouji, Toyama-shi, Toyama 930-0194 (JP); YOSHIDA Toshiko, Toyama-shi, Toyama 930-0194 (JP); OKABE Motonori, Toyama-shi, Toyama 930-0194 (JP); SOKO Chika, Toyama-shi, Toyama 930-0194 (JP); ARAKAWA Masahiko, Chikuma-shi, Nagano 387-0015 (JP)
(74) Representative: Mewburn Ellis LLP
(86) International application number: PCT/JP2020/039643
(87) International publication number: WO 2022/085138

(57) **Abstract**

The present invention addresses the problem of providing a wound contact member (or "contact layer") that directly promotes wound healing when continuously performing negative pressure wound therapy (NPWT). As a solution, there is provided dry amniotic membrane manufactured by a specified drying process, that is, raw amniotic membrane placed inside a processing tank (10) is continuously heated using an infrared heater (14) provided inside the processing tank (10) while performing a depressurization operation that places the processing tank in a depressurized state and irradiation of the raw amniotic membrane with microwaves from a microwave generating device (30) provided inside the processing tank (10) to apply energy to water molecules present inside the amniotic membrane and cause drying during a pressure recovery operation that slightly raises the pressure inside the depressurized processing tank (10) toward atmospheric pressure. Amniotic membrane, which has been dried by repeating the above process and thereby retains its cell and tissue structure, is used as a contact layer when performing negative pressure wound therapy (NPWT) on an open abdominal wound and/or wound dehiscence, which increases the healing effect of NPWT.

## Description

### Technical Field

The present invention relates to a wound contact member (or "contact layer") used when performing negative pressure wound therapy (NPWT).

### Background Art

In the field of emergency surgery, a common situation is where the abdominal wall cannot be closed due to wound dehiscence or prolonged open abdomen management due to severe abdominal trauma, peritonitis with sepsis, or the like.

Such situations involve infection, fascial necrosis, lateral involution of the abdominal wall, intestinal edema, and increased abdominal pressure.

During this time, the exposed intestinal tract forms adhesions with surrounding tissue, the intestinal tract, the abdominal wall, or the like, and forms an open wound involving intestinal exposure. In such cases, careful management is required to prevent perforation of the exposed intestinal tract, and the only option is to maintain a moist environment for the wound, avoid unnecessary irritation of the wound, and wait for the exposed intestinal tract to become covered with granulation.

Granulation will gradually grow on the exposed intestinal tract and act like the outer wall of the intestinal tract. When the wound has become covered with granulation, skin grafting and wound closure are often attempted. The granulation that grows is initially fragile and needs to be managed to avoid dryness, contact irritation, infection, and tension and/or pressure on the intestinal wall.

Perforations produced in the intestinal tract are difficult to close even when the perforated part is sutured, and form a chronic fistula. A chronic fistula like this produced on an exposed intestinal tract is referred to as an "enterroatmospheric fistula" (EAF). Although the mechanisms for the occurrence of an EAF have not been established, EAF is a serious complication that occurs for open abdominal wall wounds and wound dehiscence after trauma or peritonitis. EAF makes wound management difficult and causes a clear increase in mortality.

Negative-pressure wound therapy (hereinafter "NPWT") is a useful treatment that maintains a moist environment for wounds during management of open wounds and stimulates granulation growth. As one example, for open abdominal wall wounds, lateral involution of the abdominal wall is prevented and there is a reduction in tension that acts upon the exposed intestinal tract. However, it is not easy to completely prevent occurrence of EAF even when NPWT is used. At present, there is no established method for preventing EAF occurrence, but it is believed that early granulation grown on the exposed intestinal tract would work effectively to prevent EAF occurrence.

### Citation List

### Patent Literature

Patent Literature 1: Japanese Laid-open Patent Publication No. 2007-54015 Non Patent Literature
Non Patent Literature 1: Gruss, J. S. & Jirsch, D. W. "Human amniotic membrane: A versatile wound dressing". Can. Med. Assoc. J. 118, 1237-1246 (1978).
Non Patent Literature 2: Okabe, M. et al. "Hyperdry human amniotic membrane is useful material for tissue engineering: Physical, morphological properties, and safety as the new biological material". J. Biomed. Mater. Res. - Part A 102, 862-870 (2014).

### Summary of Invention

### Technical Problem

NPWT is a wound management method that promotes wound healing by placing a foam material, such as polyurethane, over the wound, applying a film material so as to cover the entire wound, and then continuously applying negative pressure to the covered wound.

When doing so, if there is direct contact between the foam material and the fragile wound, the resulting mechanical stimulation can cause tissue damage. For this reason, a thin dressing made of silicon or the like is often provided as a wound contact member (hereinafter simply "contact").

When performing NPWT on an open wound, a contact layer is usually laid on the bottom of the wound, a foam material is placed on top, and the wound is then sealed with a film and negative pressure is applied by a suction device. For cases where the open wound involves an exposed intestinal tract, at the present time, no contact layer is approved for use on the intestinal tract. Since applying negative pressure directly to the exposed intestinal tract by NPWT could cause perforation, strictly speaking this means that NPWT cannot be used. For this reason, the current strategy is management with gauze dressings or placing an unapproved contact layer and performing NPWT at a low pressure.

In this study, from results indicating that placing a hyper-dry amniotic membrane on an exposed intestinal tract could promote favorable growth of granulation, it was found that placing a hyper-dry amniotic membrane cut to an appropriate size on an exposed intestinal tract in an open wound, placing (any) contact layer product on top of this, and using (any) NPWT product has a merit in that it is possible to safely promote granulation on tissue on and around an exposed intestinal tract while avoiding having negative pressure act directly on the exposed intestinal tract.

Until now, no dressing material used as a contact layer has had an effect of directly promoting wound healing and/or an anti-inflammatory effect, and the main role of the material has been to simply avoid direct contact between the foam material and the wound.

### Solution to Problem

Amniotic membrane is a tough biological membrane composed of collagen and elastic fibers, and raw amniotic membrane has been reported as being a useful covering material for trauma and burns (see Non-Patent Literature 1). However, raw amniotic membrane has not been available immediately when needed and has been complicated to store and handle, which has limited its use in actual clinical practice. On the other hand, dry amniotic membrane (or "hyper-dry huma amniotic membrane": hereinafter, "HD-AM") produced by a specific drying process has also been announced (see Patent Literature 1 and Non Patent Literature 2).

The present inventors created an experimental animal model and confirmed histologically, immunochemically, and through molecular biology that HD-AM promotes granulation growth on an exposed intestinal tract. That is, the present invention uses HD-AM cells as a scaffold to induce growth factors and cell migration system chemokines, control differentiation into M2 macrophages, thereby exerting anti-inflammatory effects at a local level and promoting tissue regeneration involved.

A wound contact member according to an aspect of the present invention is a wound restoration material for use in negative pressure wound therapy, the wound contact member being a dry amniotic membrane produced by performing a drying process on raw amniotic membrane that surrounds a fetus of an animal, including a human, wherein the dry amniotic membrane has been dehydrated and dried so as to enable storage in an aseptic dry atmosphere, and amniotic membrane produced by rehydrating the dry amniotic membrane by immersion in water or a buffer retains epithelial cells, basement membrane, and connective tissue constituting the raw amniotic membrane.

By using the configuration described above, it is possible not only to prevent tissue damage due to mechanical stimulation caused by direct contact between a wound and foam material under negative pressure management but also to promote wound healing through active effects such as the wound healing promotion effect and anti-inflammatory effect of the amniotic membrane contacting the wound surface.

It is also possible for the wound contact member to be used in negative pressure wound therapy on an open abdominal wound and/or wound dehiscence.

Use of a wound contact member according to an aspect of the present invention is use of a wound restoration material for use in negative pressure wound therapy, the wound contact member including a dry amniotic membrane produced by performing a drying process on raw amniotic membrane that surrounds a fetus of an animal, including a human, wherein the dry amniotic membrane has been dehydrated and dried so as to enable storage in an aseptic dry atmosphere, and amniotic membrane produced by rehydrating the dry amniotic membrane by immersion in water or a buffer retains epithelial cells, basement membrane, and connective tissue constituting the raw amniotic membrane.

The use of a wound contact member may be use in negative pressure wound therapy on an open abdominal wound and/or wound dehiscence.

Negative pressure wound therapy according to an aspect of the present invention includes using dry amniotic membrane as a wound contact member of a wound restoration material, the dry amniotic membrane being produced by performing a drying process on raw amniotic membrane that surrounds a fetus of an animal, including a human, wherein the dry amniotic membrane has been dehydrated and dried so as to enable storage in an aseptic dry atmosphere, and amniotic membrane produced by rehydrating the dry amniotic membrane by immersion in water or a buffer retains epithelial cells, basement membrane, and connective tissue constituting the raw amniotic membrane.

The NPWT may be treatment of an open abdominal wound and/or wound dehiscence.

Note the negative pressure wound therapy NPWT referred to for the present invention can be used to treat animals, such as pets, in addition to humans.

### Advantageous Effects of Invention

By using the wound contact member according to the present invention in NPWT, it is possible to not only prevent tissue damage due to mechanical stimulation caused by direct contact between a wound and foam material under negative pressure management but to also promote wound healing through active effects, such as a wound healing promoting action and an anti-inflammatory action of the amniotic membrane in contact with the wound surface.

### Brief Description of Drawings

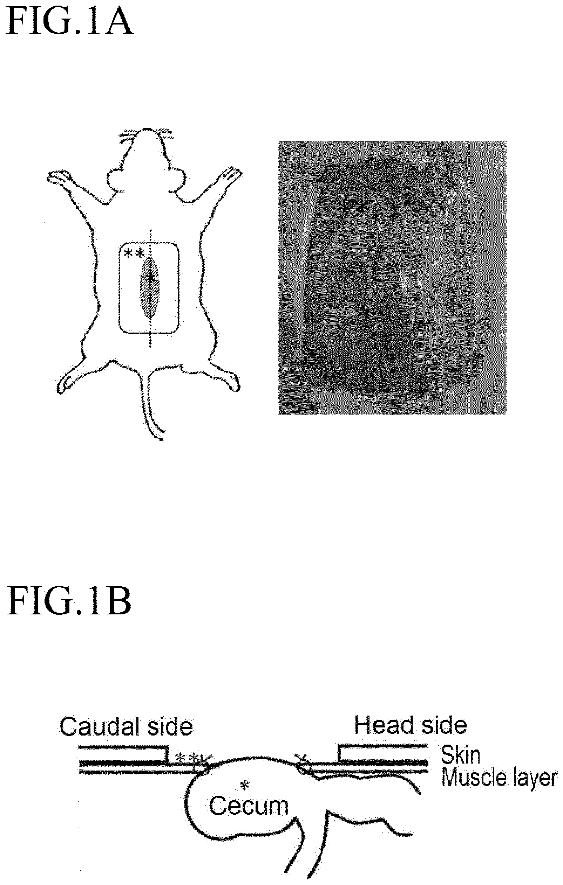

FIGS. 1A and B are diagrams useful in explaining an experimental mouse model and the application of HD-AM. In the drawing, * is the cecum, and ** the abdominal muscularis. The left part of FIG. 1A schematically shows an experimental mouse. A full-thickness skin defect was formed in the mid-abdomen with a wound size of 15 mm x 20 mm. A 15 mm incision was made over the linea alba. The right part of FIG. 1A is a photograph of the wound site of the mouse model. The cecum was lifted and fixed to the abdominal muscularis with a 6-point suture. FIG. 1B is a cross-sectional view taken along the dotted line in FIG. 1A.

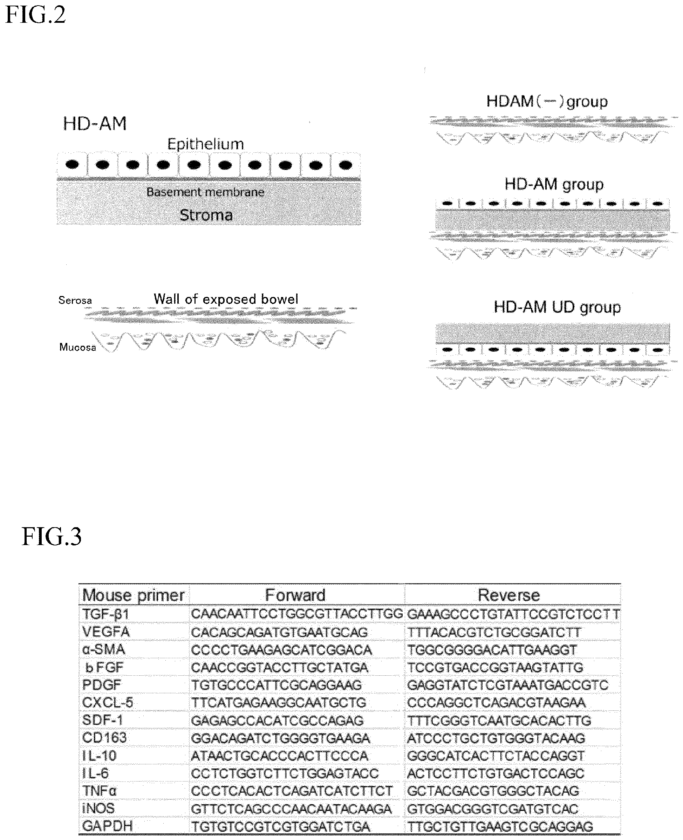

FIG. 2 is a diagram useful in explaining application of an HD-AM. The left part of FIG. 2 schematically depicts an HD-AM and the exposed intestinal wall. In the right part of FIG. 2, an HD-AM is placed on the wound area with the epithelial side facing up (HD-AM group) or the stroma side facing up (upside down, HD-AM UD group). HD-AM was not used for the control group (HD-AM(-) group).
FIG. 3 indicates primers used in quantitative reverse transcription polymerase chain reaction (qRT-PCR).

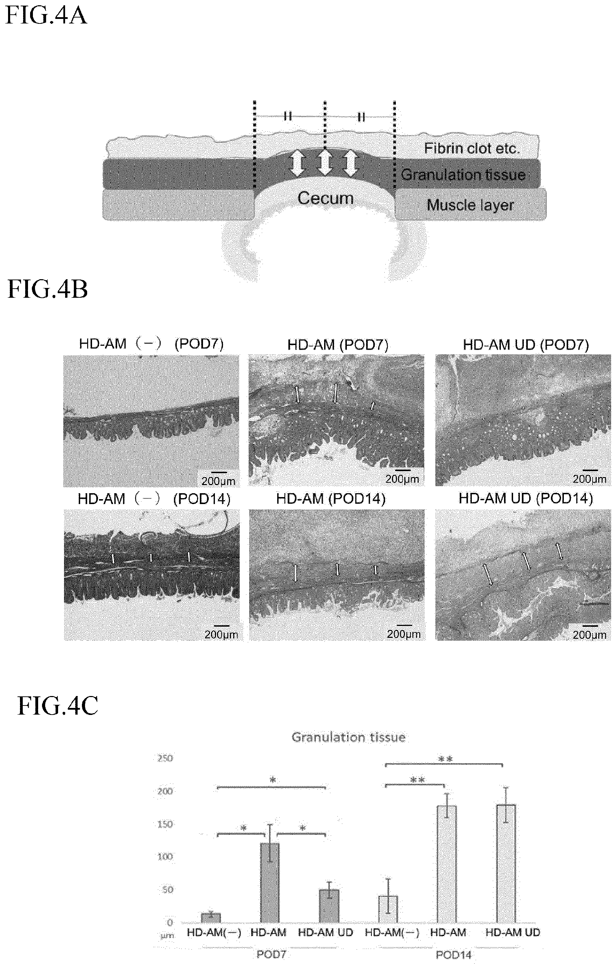

FIG. 4A schematically depicts a method for measuring the thickness of granulation tissue. Granulation tissue increases with fibrin clots in the exposed intestine. The white arrows indicate the thickness of the granulation tissue. A total of three points, including a center portion and points at 5 mm on either side of the center portion, were measured and averaged. FIG. 4B is representative photomicrographs (x50) for the respective groups. The thickness of the granulation tissue is indicated by white arrows. Since the granulation tissue is too thin, there are no arrows in the control group (POD 7). FIG. 4C is a graph comparing the measured average thicknesses of granulation tissue. The HD-AM group exhibited significantly thicker granulation tissue on both POD 7 and POD 14 compared to the control group. The thickness of the granulation tissue in the HD-AM UD group on POD 7 was significantly lower than in the HD-AM group (n=5, *p <0.05, **p<0.01).

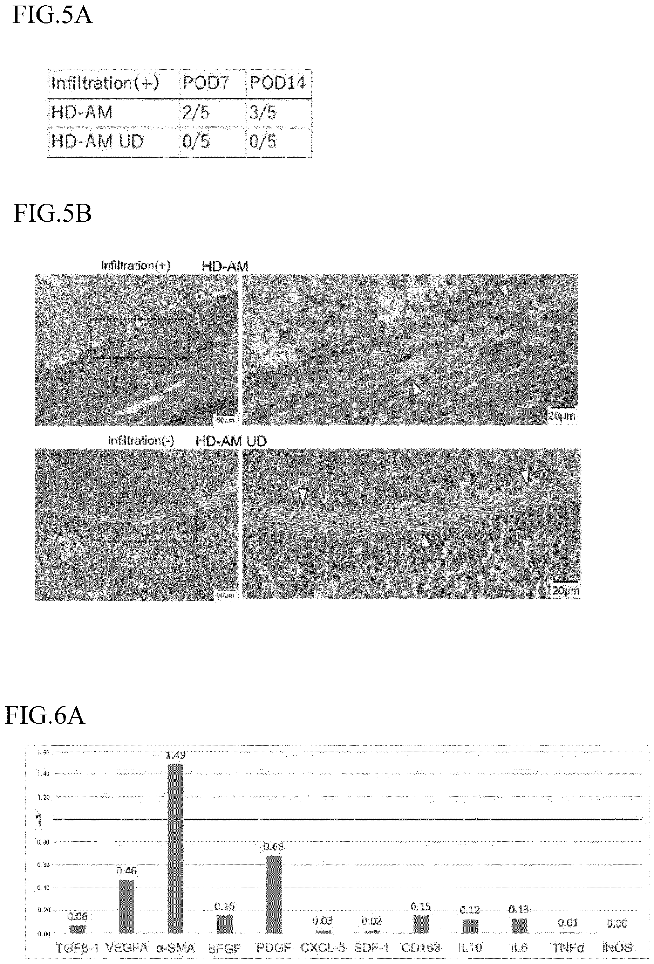

FIGS. 5A and B are a table and a diagram useful in explaining cell infiltration into HD-AM. Cell invasion into HD-AM was observed using H-E staining (x200). White in the drawing indicates HD-AM. FIG. 5A depicts the results of cell invasion in table form. Although infiltration was observed in two out of five mice on POD 7 and three out of five mice on POD 14 in the HD-AM group, no infiltration was observed in the HD-AM UD group. The upper photos in FIG. 5B indicate the observed cell infiltration into HD-AM. The lower photos in FIG. 5B indicate that no cell infiltration was observed.

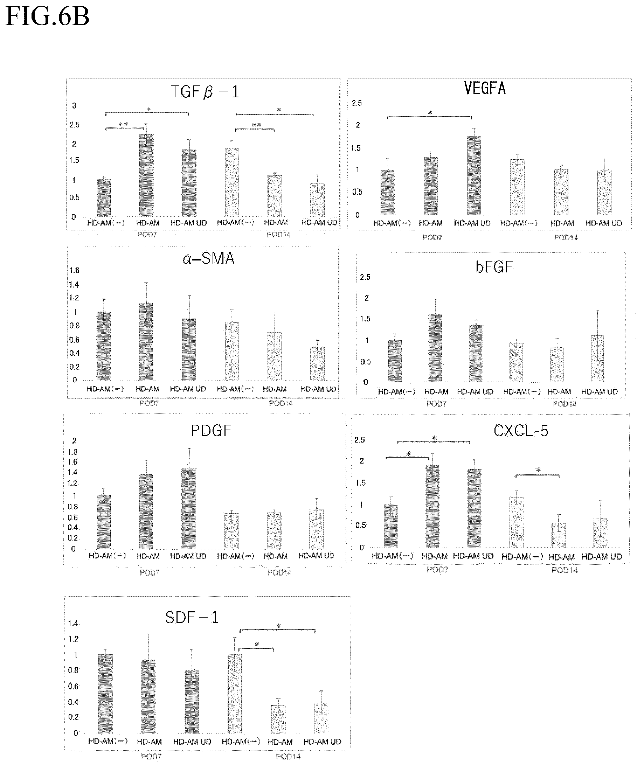

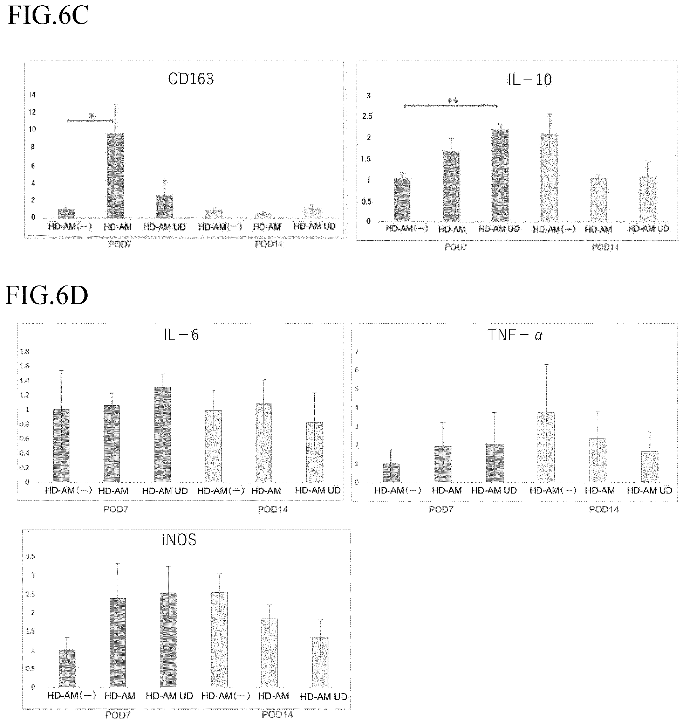

FIGS. 6A-D are graphs depicting the average relative RNA values for each group based on the POD 7 control group (n=5, *p<0.05, **p<0.01). FIG. 6A depicts that the RNA value of each primer in the HD-AM(-) group on POD 0 is 1 (solid line). Relative values on POD 0 were graphed assuming growth factors and cell migration. FIG. 6B depicts chemokines, FIG. 6C depicts anti-inflammatory markers, and FIG. 6D depicts inflammatory markers.

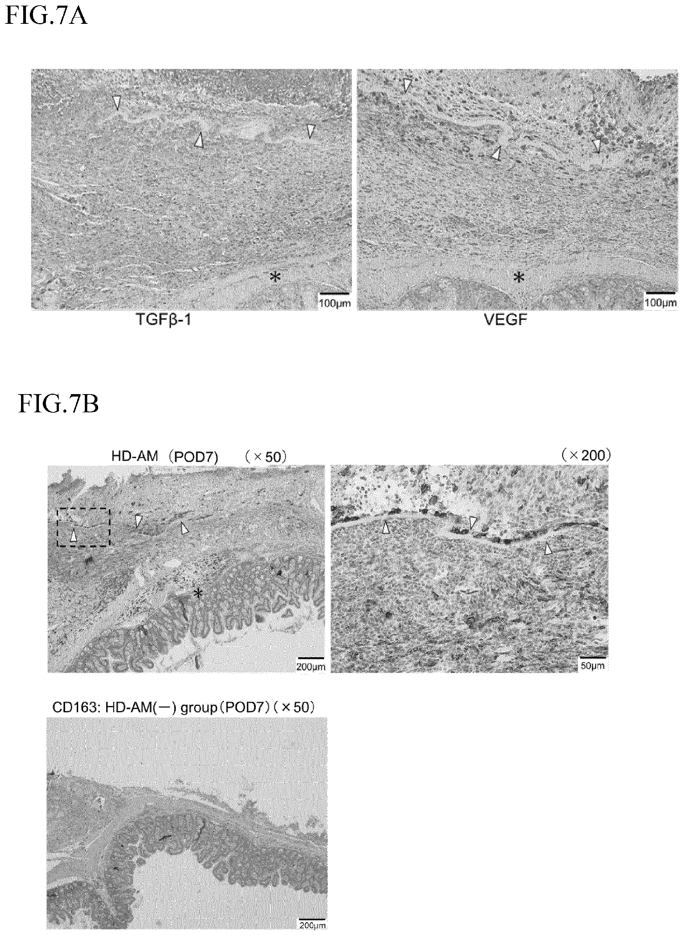

FIGS. 7A and B are photomicrographs with immunohistochemical staining. The white arrows indicate HD-AM. * is bare intestine. The left part of FIG. 7A is TGFβ-1 staining (x100), and the right part of FIG. 7A is VEGF staining (x100). The upper figures in FIG. 7B are CD163 staining. The focal point (x50) framed by a dotted line on the upper left photograph of FIG. 7B is enlarged and displayed in the upper right photograph (x200) of FIG. 7B. The epithelial side of HD-AM faces upward and the stromal side faces downward. The bottom figure in FIG. 7B is CD163 staining of the control (x50).

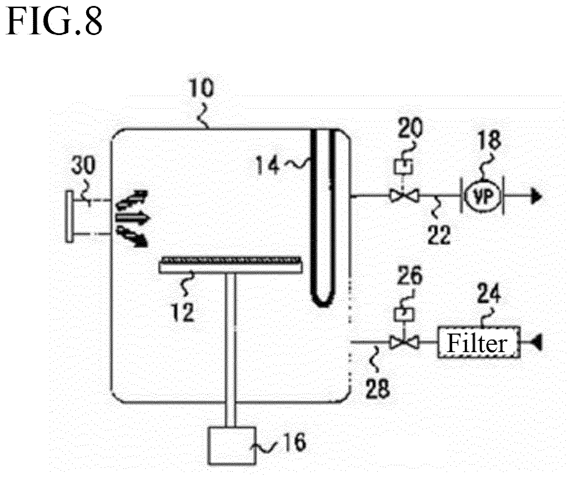

FIG. 8 depicts a drying device for preparing an HD amniotic membrane.

### Description of Embodiments

### Overview of Embodiments

A dried amniotic membrane manufactured by a specified drying process (called "hyperdrying") is, for example, the dried amniotic membrane described in Patent Literature 1. That is, raw amniotic membrane placed in a processing tank is continuously heated by a far-infrared heater provided inside the processing tank, and a depressurization operation, in which the inside of the processing tank is placed in a depressurized state, and irradiation of the raw amniotic membrane with microwaves from a microwave generating device provided inside the processing tank to apply energy to water molecules present inside the amniotic membrane and cause drying during a pressure recovery operation that slightly raises the pressure inside the depressurized processing tank toward atmospheric pressure were performed. In dried amniotic membrane (HD-AM) manufactured by repeating the above process a plurality of times, the amniotic membrane cells themselves are inactivated but the cell and tissue structures are retained.

In typical NPWT, (1) a foam material (or "covering material") such as polyurethane is applied in keeping with the shape of the wound and is sealed with transparent film. (2) A drainage tube is connected to the foam material through a hole provided in the transparent film. (3) The drainage tube is connected to a suction source and is adjusted to close the open wound, while at the same time removing excess fluid from the wound bed to facilitate circulation and drainage of exudate. This creates a moist treatment environment and prevents swelling.

In this embodiment, HD-AM is disposed as a wound contact member (or "contact layer"). By disposing HD-AM as a wound contact member between the wound and the foam material, damage to tissue due to mechanical irritation caused by direct contact between the foam material and the fragile wound is prevented and active effects of the amniotic membrane that contacts the wound surface, such as a wound healing promoting action and an anti-inflammatory action, are promoted.

In the present embodiment, when HD-AM is used as a contact layer, as one example, the HD-AM may be formed into an appropriate shape with scissors or the like in keeping with the wound location, such as an open abdominal wound, and a drainage hole may be fabricated. Also, by simply placing HD-AM with the epithelial side on top and the stromal side facing the wound surface, it is possible to take advantage of the anti-inflammatory effect and wound healing promoting effect of the amniotic membrane as a scaffold.

### Creation of Open Wounds in Model Animals

An 8-week-old male ICR mouse was anesthetized and placed in the supine position before the four limbs were fixed. A 15 x 20 mm (horizontal x vertical) section of skin of the abdominal wall was removed to expose the abdominal wall sarcolemma, and a 15 mm midline incision was made in the abdominal wall to identify the cecum (see FIG. 1A). The cecum was then raised and a 6-needle fixation of the serosal and abdominal wall muscularis of the cecum was made using 7-0 nylon thread (see FIG. 1B) to create a fragile open wound with an exposed intestinal tract.

Note that the handling of laboratory animals was approved by the Institutional Animal Care and Use Committee of the University of Toyama in accordance with the guidelines of the National Institutes of Health. The experiments were conducted according to the guidelines of the Institutional Animal Care and Use Committee of the University of Toyama.

### Application of HD-AM

The human amniotic membrane is mainly composed of three layers, a monolayer epithelial layer (hereinafter "epithelium"), a thin basement membrane (hereinafter "basement membrane"), and an avascular stromal layer (hereinafter "stroma") (see the left part of FIG. 2). In the experiment, the HD-AM was cut into 15 x 20 mm sizes and three groups (see the right part of FIG. 2) were formed, where specimens with amniotic membrane were split according to front and rear into a group with the epithelium facing up (hereinafter "HD-AM group") and a group with the stroma facing up (hereinafter "HD-AM UD" group), and a group where no HD-AM was placed as a control (hereinafter "HD-AM(-) group"). The wounds (or " wall of exposed bowel) were all covered with Hydrosite plus (made by Smith & Nephew Wound Management, a dressing with a three-layer structure composed of a non-adhesive wound contact layer made of polyurethane, an absorbent pad of hydrophilic foam, and a backing film).

Each of the groups ("HD-AM(-)", "HD-AM", and "HD-AM UD") contained five mice which were evaluated 7 days post operation (POD 7) and 14 days post operation (POD 14), meaning a total of thirty animals were used. The wounds were covered with stainless steel mesh (0.06 mmΦ, 150 m/s) to prevent deviation of the HD-AM and/or wound covering due to mouse behavior.

### Histological and Immunohistochemical staining

To perform histological observation, the abdominal wall was collected from each mouse as the exposed intestinal tract on POD 7 and POD 14 and embedded in paraffin. Sections sliced from the paraffin block were stained with hematoxylin-eosin (H&E) and Azan. In addition, immunohistochemical staining was also performed for CD163, TGFβ-1 (transforming growth factor beta-1), and VEGF (vascular endothelial growth factor). Stained tissue was imaged using a Leica DMRBE microscope (Leica, Wetzlar, Germany) and a DP73 system (Olympus, Tokyo, Japan).

### Measurement of Granulation Tissue on Exposed Intestine

Azan staining facilitates the distinction between collagen fibers and fibrin for observation of granulation tissue. The thickness of granulation tissue containing collagen fibers was measured using the Olympus CellSens imaging program (version 1.7; Olympus, Tokyo, Japan). The measurement sites were the center of the positions where the exposed intestinal tract was fixed to the left and right muscularis, and 5mm on both sides from the center, and an average value of the three measured sites was calculated (see FIG. 2A).

### Quantitative Reverse Transcription Polymerase Chain Reaction (qRT-PCR)

To extract mRNA of granulation tissue on the exposed intestinal tract, target sites were selectively collected from each specimen. During collection, granulation tissue was dissected in the same way for each specimen in a central part of the exposed intestinal tract to make the samples as anatomically constant as possible and avoid any influence from differences in the collection site or range. Total mRNA was extracted from the tissue using Isogen II (Nippon Gene Co. LTD., Tokyo, Japan) according to the manufacturer's instructions. A 3 µg portion of each mRNA was treated with deoxyribonuclease I (DNase I, made by Sigma-Aldrich, Inc., Tokyo, Japan) for 15 minutes at room temperature.

cDNA was synthesized using 500 ng of DNase I-treated RNA using the Rever Tra Ace qPCR RT Kit (made by Toyobo Co., Ltd., Osaka, Japan). Gene expression was subjected to real-time RT-PCR analysis using Brilliant SYBR Green QRT-PCR Mix (Stratagene; Agilent Technologies Japan Ltd. Japan) using an Mx3000P quantitative polymerase chain reaction (qPCR) system (Stratagene; Agilent Technologies Japan Ltd, Japan). On POD 7 and POD 14, mRNA was extracted from each of five mice in each group, and the growth factors TGFβ130, VEGFA31, α-SMA32, bFGF33, and PDGF34, the cell migration chemokines CXCL-535 and SDF-135, CD163 which is a marker for anti-inflammatory M2 macrophages, the anti-inflammatory cytokine IL-1037, the inflammatory cytokine IL-637, tumor necrosis factor-alpha (TNF-α38), and inducible nitric oxide synthase (iNOS38) were measured in triplicate.

To establish that the variations in cytokines were postoperative changes, tissue was collected immediately after creating the five model mice, and the mRNA expression level of each primer immediately after the operation was examined as POD 0. The expression level of each mRNA was corrected with GAPDH as an internal control, and relative comparisons were made based on the mean value of the HD-AM(-) group on POD 7. The sequence of the primers used here is depicted in FIG. 3.

### Evaluating the Thickness of Exposed Intestinal Granulation Tissue

The thickness of granulation tissue on the exposed intestinal tract was measured in each group and compared on POD 7 and POD 14.

The HD-AM and HD-AM UD groups had significantly thicker granulation tissue on both POD 7 and POD 14 than the HD-AM(-) group (p< 0.05 and p< 0.01). On the other hand, the granulation tissue was significantly thicker in the HD-AM group than in the HD-AM UD group (p< 0.05) on POD 7, with similar levels on POD 14 (see FIGS. 4A to 4C).

### Cell Infiltration into HD-AM

Cell infiltration into the HD-AM was observed for the HD-AM group. However, it was not observed for the HD-AM UD group. Cell infiltration was observed at a rate of 5/10 for HD-AM 5/10 and 0/10 for HD-AM UD, and was therefore significantly higher for the HD-AM group (p< 0.05). (see FIGS. 5A and 5B).

### Evaluation of Expressions on POD 0 Compared to HD-AM(-) group on POD 7

Cytokines aside from VEGFA, α-SMA, and PDGF were hardly expressed immediately after the operation (POD0) (see FIG. 6A).

### Growth factors and Cell Migration Chemokines

Expression of TGFβ-1 was significantly higher in the HD-AM and HD-AM UD groups on POD 7 than the HD-AM(-) group, but this was reversed for both on POD 14. In the same way as TGFβ1, CXCL-5 was significantly higher in the HD-AM group than the HD-AM(-) group on POD 7, but this was also reversed on POD 14. VEGF was significantly higher in only the HD-AM UD group on POD 7 than the HD-AM(-) group, and SDF-1 expression on POD 14 was significantly lower for the HD-AM group and HD-AM UD group than the HD-AM(-) group. No significant differences were observed for other growth factors (see FIG. 6B).

### Anti-inflammatory Markers

CD163 was expressed significantly higher on POD 7 in the HD-AM group than the HD-AM(-) group. IL-10 was expressed significantly higher on POD 7 in the HD-AM UD group than the HD-AM(-) group. Although there was no significant difference, the HD-AM group tended to be higher than the HD-AM(-) group on POD7 (p = 0.057) (see FIG. 6C).

### Inflammation Markers

When the respective groups were compared, no significant differences were observed in the expression of IL-6, TNF-α, and iNOS (see FIG. 6D).

### (Immunohistochemical Staining)

Localization of TGFβ-1, VEGF, and CD163 was investigated by immunohistochemical staining. TGFβ-1 and VEGF were highly expressed in exudate from the wound and in the periphery of the amniotic membrane (see FIG. 7A). Notably, on POD7, CD 163 was expressed so as to be concentrated on the epithelium side of the HD-AM (see the arrows in FIG. 7B).

### Manufacture of HD-AM

Using the drying devices depicted in FIG. 8, a raw amniotic membrane was dried with vacuum, far-infrared, and microwave devices set at the conditions given below.

The drying device in FIG. 8 is equipped, inside a processing tank 10, with a rotary table 12 on which raw amniotic membrane is placed, and a far-infrared heater 14 as a heating means. Also, to reduce the pressure inside the processing tank 10, a solenoid valve 20 and a vacuum pump 18 are connected to the processing tank 10. Also, to repressurize the processing tank 10 interior, a solenoid valve 26 and a filter 24 are connected to the processing tank 10.

The treatment tank 10 is also provided with a microwave irradiation device 30 which performs, when a pressure recovery operation has been performed from a reduced pressure state, drying while applying energy to water molecules in the amniotic membrane

Drying tank heating temperature: 50°C, FIR: 50°C, Stop valve: 37%, Maximum ultimate pressure 0.34kPa Dry running maximum ultimate pressure 0.33kPa

### Drying method

(1) Depressurization 180sec
(2) Pressure recovery 30 sec (with stop valve 37% open)
   Commence emission of microwaves
   0.1kw for 180sec (pressure recovery continues)
(3) Depressurization 180 sec
(4) Repeat (2) and (3) thereafter
(5) End drying manually by checking the ultimate pressure after 180 sec of depressurization in (3) is (0.30 to 0.35 kPa).

Process complete when atmospheric pressure is restored

### Industrial Applicability

Dried amniotic membrane (HD-AM) is manufactured by a specific drying process, that is, raw amniotic membrane that has been placed inside a processing tank is continuously heated by an infrared heater provided inside the processing tank while performing a depressurization operation that reduces the pressure inside the processing tank and irradiation of the raw amniotic membrane with microwaves from a microwave generating device provided inside the processing tank to apply energy to the water molecules present inside the amniotic membrane and dry the amniotic membrane during a pressure recovery operation that slightly raises the pressure inside the depressurized processing tank toward atmospheric pressure were performed. By repeating the above multiple times, it is possible to improve storage stability and handling of the dried amniotic membrane while preserving the cell and tissue structure. By using this dry amniotic membrane as a contact layer to prevent contact between the dressing foam and the wound surface during negative pressure wound therapy (NPWT) on open abdominal wounds and/or wound dehiscence, the healing effect of NPWT can be enhanced.

## Claims

1. A wound contact member that is a wound restoration material for use in negative pressure wound therapy,
the wound contact member comprising a dry amniotic membrane produced by performing a drying process on raw amniotic membrane that surrounds a fetus of an animal, including a human,
wherein the dry amniotic membrane has been dehydrated and dried so as to enable storage in an aseptic dry atmosphere, and
amniotic membrane produced by rehydrating the dry amniotic membrane by immersion in water or a buffer retains epithelial cells, basement membrane, and connective tissue constituting the raw amniotic membrane.

2. The wound contact member according to claim 1,
wherein the wound contact member is used in negative pressure wound therapy on an open abdominal wound and/or wound dehiscence.

3. Use of a wound contact member that is use of a wound restoration material for use in negative pressure wound therapy,
the wound contact member including a dry amniotic membrane produced by performing a drying process on raw amniotic membrane that surrounds a fetus of an animal, including a human,
wherein the dry amniotic membrane has been dehydrated and dried so as to enable storage in an aseptic dry atmosphere, and
amniotic membrane produced by rehydrating the dry amniotic membrane by immersion in water or a buffer retains epithelial cells, basement membrane, and connective tissue constituting the raw amniotic membrane.

4. The use of a wound contact member according to claim 3 that is use in negative pressure wound therapy on an open abdominal wound and/or wound dehiscence.

5. Negative pressure wound therapy comprising using dry amniotic membrane as a wound contact member of a wound restoration material, the dry amniotic membrane being produced by performing a drying process on raw amniotic membrane that surrounds a fetus of an animal, including a human, wherein the dry amniotic membrane has been dehydrated and dried so as to enable storage in an aseptic dry atmosphere, and amniotic membrane produced by rehydrating the dry amniotic membrane by immersion in water or a buffer retains epithelial cells, basement membrane, and connective tissue constituting the raw amniotic membrane.

6. The negative pressure wound therapy according to claim 5 as a treatment of an open abdominal wound and/or wound dehiscence.
